# EUROPEAN PATENT SPECIFICATION

(11) **EP 3 858 362 B1**
(45) Date of publication and mention of the grant of the patent: **22.07.2026**
(21) Application number: 20154270.1
(22) Date of filing: 29.01.2020
(51) Int. Cl.: A61K 31/716, A61P 37/04

(54) **BETA-GLUCAN FOR USE IN THE TREATMANE OF A REMISSION**
BETA-GLUCAN ZUR VERWENDUNG BEI DER BEHANDLUNG EINER REMISSION
BÊTA-GLUCANE UTILISÉ POUR LE TRAITEMENT D'UNE RÉMISSION

(43) Date of publication of application: 04.08.2021
(73) Proprietor: Pleuran, s.r.o., 821 05 Bratislava (SK)
(72) Inventor: Spacek, Ján, 169 00 Praha (CZ); Závadová, Eva, 160 00 Praha (CZ); Konopásek, Bohuslav, 142 00 Praha (CZ); Gabriz, Ján, 821 01 Bratislava (SK); Polák, Martin, 84105 Bratislava (SK)
(74) Representative: Porubcan, Róbert

(56) References cited:
- WO-A1-2012/060783
- WO-A2-02/47612
- WO-A2-2008/057501
- TAGUCHI T ET AL: "Life span prolongation effect of lentinan on patients with advanced or recurrent colorectal cancer", INTERNATIONAL JOURNAL OF IMMUNOPHARMACOLOGY, ELMSFORD,NY, US, vol. 4, no. 4, 1 January 1982 (1982-01-01), pages 271, XP023823279, ISSN: 0192-0561, [retrieved on 19820101], DOI: 10.1016/0192-0561(82)90140-0
- JINN-CHYI WANG ET AL: "Optimization for the production of water-soluble polysaccharide from Pleurotus citrinopileatus in submerged culture and its antitumor effect", APPLIED MICROBIOLOGY AND BIOTECHNOLOGY, SPRINGER, BERLIN, DE, vol. 67, no. 6, 1 June 2005 (2005-06-01), pages 759 - 766, XP019331866, ISSN: 1432-0614, DOI: 10.1007/S00253-004-1833-X
- JESENAK MILOS ET AL: "Immunomodulatory effect of pleuran ([beta]-glucan fromPleurotus ostreatus) in children with recurrent respiratory tract infect", INTERNATIONAL IMMUNOPHARMACOLOGY, ELSEVIER, AMSTERDAM, NL, vol. 15, no. 2, 20 December 2012 (2012-12-20), pages 395 - 399, XP028989510, ISSN: 1567-5769, DOI: 10.1016/J.INTIMP.2012.11.020
- JESENAK MILOS ET AL: "Immunomodulatory effect of pleuran ([beta]-glucan fromPleurotus ostreatus) in children with recurrent respiratory tract infect", INTERNATIONAL IMMUNOPHARMACOLOGY, ELSEVIER, AMSTERDAM, NL, vol. 15, no. 2, 20 December 2012 (2012-12-20), pages 395 - 399, XP028989510, ISSN: 1567-5769, DOI: 10.1016/J.INTIMP.2012.11.020
- TAGUCHI T ET AL: "Life span prolongation effect of lentinan on patients with advanced or recurrent colorectal cancer", INTERNATIONAL JOURNAL OF IMMUNOPHARMACOLOGY, ELMSFORD,NY, US, vol. 4, no. 4, 1 January 1982 (1982-01-01), pages 271, XP023823279, ISSN: 0192-0561, [retrieved on 19820101], DOI: 10.1016/0192-0561(82)90140-0

## Description

### Field of technology

The invention concerns the use of beta-glucans, mainly fungal β (1,3/1,6) glucan (β-(1→3), (1-6)-glucan), in remission after oncological treatment of the immunosensitive breast cancer. The increase in anti-tumor cellular immunity has been reliably observed during the peroral long-term use of fungal β (1,3/1,6) glucan.

### Prior state of the art

Anti-tumor cellular immunity is based on T lymphocyte activation and subsequent recognition of tumor antigens. Tumor-transformed cells originate in tissues and they are eliminated by T lymphocytes. The presence of CD 8+ cytotoxic lymphocytes in the tumor microenvironment is considered to be a significant positive prognostic factor in patients with locally advanced colorectal cancer (Galon, Pages, et al. 2018).

Publication US2015064199 A1 discloses the method of use of neutral dissolved glucan and monoclonal antibodies for anti-tumor therapy. Neutrally dissolved β (1,3/1,6) glucan increases the tumoricidal activity of the innate immune system by binding the C3 complement to the CR3 protein receptor.

Publication US2001043914 A1 tackles the prevention and treatment of the tumors by use of slow-releasing microparticles containing IL-12 which are injected directly to the tumor.

Publication EP1651676 B1 discloses the method of implementation of substances to the cells by means of orally administered beta-glucan.

Publication US2010/0166751 A1 discloses the use of beta-glucan for suppression and elimination of tumor cells in primary treatment. Beta-glucan pursuant to this publication is isolated from the cell walls of the yeasts. The use of beta-glucan in the primary treatment does not have convincing and verified results, yet.

Publication WO2008/057501 A2 describes β-I-6-glucans, compositions and devices comprising the same, and methods of use thereof in modulating immune responses. The β-I-6-glucans are enriched for O-acetylated groups and/or conjugated to a solid support or linked to a targeting moiety.

An increase of the anti-tumor cell immunity in the remission phase after primary treatment is desired, whereby the mean for increase of such anti-tumor immunity should not cause, even after long-term use, the worsening of organ functions, mainly functioning of liver, kidneys, and so on.

### Essence of the invention

The invention is as defined in claims 1 to 8. The abovementioned deficiencies are significantly remedied by a beta-glucan-based polysaccharide immunomodulator which is used for increase of the anti-tumor cell immunity in remission after primary treatment of solid tumors. The primary treatment can include surgery and/or radiotherapy and/or chemotherapy and/or hormonal therapy and/or targeted biological therapy. Effective use of the beta-glucan in remission is proved irrespective of the type of primary oncological treatment; whereby significant results were recorded in cases of immunosensitive cancer such as breast cancer. In cases of these widespread oncological diagnoses the proofs of the significance and interpretation of the tumor-infiltrating lymphocytes are, in general, accepted. These are malignant diseases where one can expect the overall beneficial contribution of the immunomodulation in sense of stimulation of the cellular immunity response.

Significant preventive and therapeutic effects during the continuous long-term use of beta-glucan in remission during the treatment of breast cancer, follow from the clinical study; the concentration of CD8+ lymphocytes increased alongside subsequent increase of CD19+ lymphocytes.

The term "increase of anti-tumor immunity" in this text denotes any change of the physiological rations of the cells in such a way that it increases the ability to eliminate the tumor-transformed cells. This increase of anti-tumor immunity is usually accompanied by increased concentration of CD8+ lymphocytes, CD19+ lymphocytes, increase of IgG3, IgA, CD16+56+, whereby the demarcation of boundary where the process is preventive and where is it therapeutic is not important.

Contrary to the methods known in the prior art, the beta-glucan according to this invention is used for long term and perorally, which can be easily ensured if the patient cooperates. Clinical study has shown a preventive anti-cancer effect during the long-term use without undesired side effects during simultaneous decrease in the incidence of the common infections.

First significant changes have been recorded after 3 months in the humoral component of the immune system; these changes appeared as significant increase of B lymphocytes and immunoglobulin. First significant changes in the cellular immunity (NK cells and CD8+ lymphocytes) took place only after longer period of use of beta-glucan. Immunomodulation by means of beta-glucan is therefore, pursuant to this invention, recommended in the adjuvant mode. Immunity response takes place with a longer lag after the initiation of therapy; significant improvement of the immunological parameters takes place 12 to 15 months after the beginning of the use, which is why beta-glucan is suitable for long-term administration.

The result of the invention is clear effect of the perorally used preparation on the support of the immune system of the individual in period of so-called complete remission. Significant adjustments of the physiological ratios of the cellular anti-tumor immunity during continuous use of the beta-glucan serve the purpose of restoration of the immune system and alleviation of secondary immunodeficiency in cancer patients, e.g. in patients with hormone-dependent breast cancer.

Beta-glucan as a preparation according to this invention is in the preferably arrangement a fungal β (1,3/1,6) glucan. Such beta-glucan can be prepared from the oyster mushroom (Pleurotus ostreatus), when such beta-glucan is also called pleuran.

The subject matter of the protection is also a composition which includes beta-glucan according to the description, whereby it is adapted for the oral use, for example, in form of capsules. The composition can - aside from beta-glucan - also include vitamin C and, possibly, some bacterial cultures, oligosaccharides, adjuvants, conditioning agents such as preservatives, and so on.

Beta-glucan is administered to patients which are in remission at least for 6 months - that is, it is administered after 6 months since the conclusion of the primary oncological treatment at earliest; preferably after 12 months since the conclusion of the primary oncological treatment at earliest.

The dosage regimen (regime of dosing, dosage regime) of beta-glucan is also subject of protection, whereby this dosage regimen demonstrably leads to effective results. The essence of the sequential dosage regimen is the alteration of repeated phases. Beta-glucan is used continually, without a stage of zero dosage, in two subsequent and subsequently repeated phases, whereby during the first phase a high dose of beta-glucan is used and in the second phase a low dose of beta-glucan is used. These two phases are subsequently altered, therefore the names "first" and "second" are only used to distinguish them. High dose of beta-glucan is at least twice the low dose. High dose of beta-glucan can range from 600 mg to 800 mg, preferably 700 mg; low dose of beta-glucan can range from 50 mg to 300 mg, preferably 100 mg to 200 mg.

Pursuant to the results of clinical study it is preferable if the high dose has constant level during the use and the low dose regularly alters between values 100 mg and 200 mg. First and second phase can last for the same period of time, that is, 2 to 4 months, preferably 3 months.

Beta-glucan (polysaccharide beta-glucan-based immunomodulator) brings, during its long-term use, significant improvement of all crucial oncomarkers (tumor markers); it is manifested as significant preventive preparation, whereby no patient from the assessed group has fallen into relapse. The advantage of beta-glucan is an absence of the undesired side effects. On contrary, the administration of beta-glucan leads to positive side effects in the immunological picture of patients.

### Description of drawings

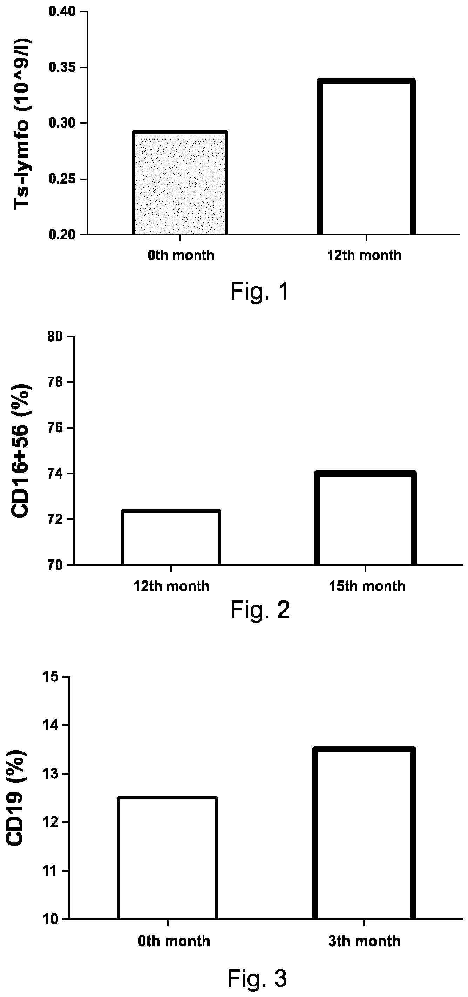

Results and effects of the invention are further disclosed by means of figures 1 to 6.

Graph Ts-lymfo on figure 1 shows a significant increase of the concentration of the absolute number of CD8+ (cytotoxic T lymphocytes) during 12 months after the start of the clinical test in the regime of sequential dosage with repeated 700mg-100mg-700mg-200mg/day phases in case of group of patients using β (1,3/1,6) glucan (p=0,0147). The value for the 12^{th} month corresponds to end of 700mg-100mg-700mg-200mg/day dosage.

Graph CD16+56+ on figure 2 shows a significant increase of the percentual share of CD16+56+ (NK calls) in 15 months after the start of the clinical test in the regime of sequential dosage with repeated 700mg-100mg-700mg-200mg/day phases in case of group of patients using β (1,3/1,6) glucan (p=0,0313). The value for the 15^{th} month corresponds to state at the end of 700mg/day dose.

Graph CD19+ on figure 3 depicts the comparison of the percentual share of B lymphocytes at the beginning of the use (0 months) and for period of 3 months at dosage 700mg/day. Significant increase of the percentual share of CD19+ took place after 3 months of usage of daily dose of 700mg/day (p=0,0246). The value displayed for the 3^{rd} month corresponds to state at the end of 700mg/day dose.

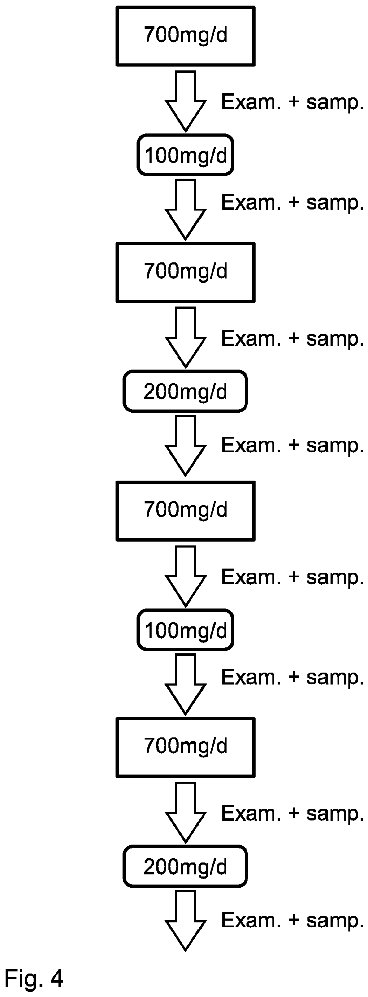

Figure 4 depicts the sequential dosage regimen. The shortcut "Exam." refers to patient examination; the shortcut "samp." refers to sampling.

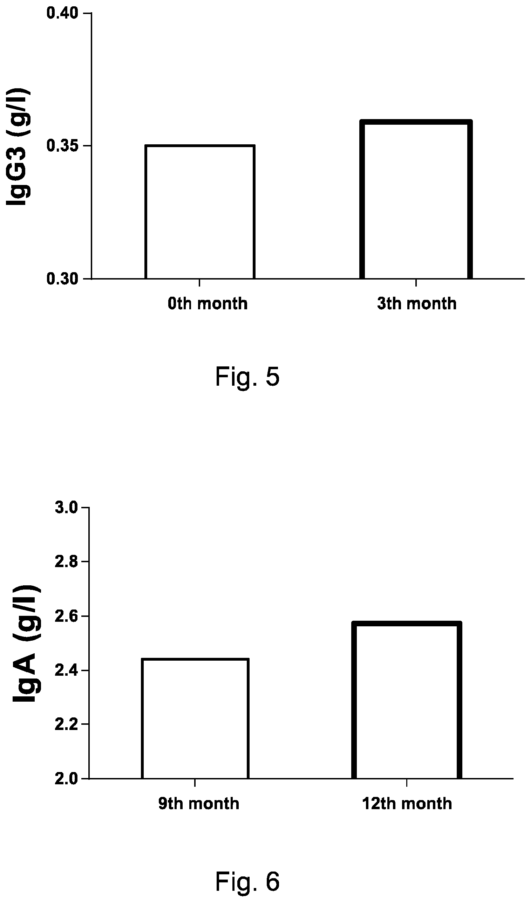

Graph IgG3 on figure 5 is a comparison of the concentration of IgG3 at the start of the use (0 months) with the concentration of IgG3 after 3 months of use of β (1,3/1,6) glucan at dosage 700mg/day. Graph shows a significant increase in the concentration of IgG3 after 3 months of use (p=0,0010). The value displayed for 3^{rd} month corresponds to state at the end of 700mg/day dose.

Graph IgA on figure 6 is a comparison of the concentration of IgA after the conclusion of sequence with high dose 700mg/day in 9^{th} month and subsequently after three further months of use with low dose of β (1,3/1,6) glucan at 200mg/day. The graph shows significant increase of the IgA concentration after 3 months of use, in total for 12 months since the start of the study (p=0,0255). The value displayed for 12^{th} month corresponds to state at the end of 200mg/day dose.

### Example of realization

In a clinical study with the results pursuant to figures 1 to 6 the purpose was to determine the way in which immune system of the patients with hormonal-dependent breast cancer reacts to the continual use of β (1,3/1,6) glucan in clinical and display remission.

Clinical study involved 60 patients who underwent standard oncological treatment for the indication of locally advanced hormonal-dependent breast cancer of I-II clinical stage. The primary treatment of the cancer in the group involved various methods: surgery, radiotherapy, chemotherapy, hormonal therapy, pursuant to the individual indication. Before their inclusion in the clinical study the patients were examined by the clinical immunologist in order to rule out immunopathology and allergic or autoimmune underlying diseases. The anti-tumor immunity (CD4+, CD8+, B cells) were examined by a method of flow cytometry. TGF beta and VEGF were measured by ELISA method.

The patients used β (1,3/1,6) glucan perorally in a sequential regime, whereby they altered phases with high doses with phases with low doses, whereby the use was continuous without the zero dose phases. Beta-glucan (named "Imunoglukan", which is the invention applicant's trademark) was in form of capsules which also included calcium L-ascorbate, magnesium stearate and benzoic acid.

Significant increase of B lymphocytes (CD19+) took place after three months of use of β (1,3/1,6) glucan with dosage 700mg/day (p=0,0246). After three months of use a significant increase of level of IgG3 (p=0,0387) was recorded, too.

Significant increase of level of IgA took place after 12 months since the start of the study (p=0,0255).

Significant increase of CD16+56+ level took place after three months after repeated increase of the doses from 200mg/day to 700mg/day (15 months since the start of the study) (p=0,0469).

After 15 months since the start of the study a significant increase of concentration of CD8+ (cytotoxic T lymphocytes) at dosage regimen 700mg-100mg-700mg-200mg/day took place (p=0,0490).

During the long-term administration of β (1,3/1,6) glucan there were no changes in liver tests, renal function and autoimmune parameters. Clinically, Imunoglukan was very well tolerated by the patients and a decrease in incidence of infections (URT, uroinfections, gynecological infections, herpes) took place.

No patient from the observed group in the remission entered a relapse. The results of the clinical study pursuant to figures 1 to 6 also show the increase of the effects during sequential dosage while the continuous use is ensured.

The references to methods of treatment by therapy in this example are to be interpreted as references to medicaments of the present invention for use in those methods.

### Industrial applicability

Industrial applicability of this invention is obvious. According to this invention it is possible to industrially and repeatedly produce a preparation, or composition, respectively, for increase of the anti-tumor immunity in remission after primary treatment of the breast cancer.

## Claims

1. Beta-glucan for use in the treatment of a remission after treatment of hormone-dependent breast cancer, in a form prepared for oral administration, administered after 6 months since a conclusion of a primary oncological treatment at earliest, for a continuous use in two subsequent and subsequently repeated phases, whereby during the first phase a high dose of beta-glucan is used and in the second phase a low dose of beta-glucan is used, whereby the high dose of beta-glucan is at least twice the low dose of beta-glucan and low dose is non-zero, the first phase and the second phase last 2 to 4 months.

2. Beta-glucan for the use according to the claim 1 **characterized by the fact**, that it is fungal β (1,3/1,6) glucan.

3. Beta-glucan for the use according to the claim 2 **characterized by the fact**, that it is prepared from an oyster mushroom.

4. Beta-glucan for the use according to any of the claims 1 to 3, administered after 12 months since the conclusion of the primary oncological treatment at earliest.

5. Beta-glucan for use according to the claims 1 to 4 is **characterized by the fact**, that the high dose of beta-glucan ranges daily from 600 mg to 800 mg, preferably 700 mg, and the low dose of beta-glucan ranges daily from 50 mg to 300 mg, preferably 100 mg to 200 mg.

6. Beta-glucan for use according to the claims 1 to 5 is **characterized by the fact**, that the high dose has a constant level during the use and the low dose regularly alternates between values 100 and 200 mg.

7. Beta-glucan for use according any of the claims 1 to 6 is **characterized by the fact**, that the first phase and the second phase last 3 months.

8. Beta-glucan for use according any of the claims 1 to 7 is **characterized by the fact**, that the first phase and the second phase have identical temporal duration.

## Patentansprüche

1. Beta-Glucan zur Anwendung bei der Behandlung in Remission nach der Therapie eines hormonabhängigen Brustkarzinoms, in einer für die orale Verabreichung geeigneten Form, verabreicht frühestens 6 Monate nach Abschluss der primären onkologischen Behandlung, zur kontinuierlichen Einnahme in zwei aufeinanderfolgenden und anschließend wiederholten Phasen, wobei während der ersten Phase eine hohe Dosis Beta-Glucan und in der zweiten Phase eine niedrige Dosis Beta-Glucan verabreicht wird, wobei die hohe Dosis Beta-Glucan mindestens doppelt so hoch ist wie die niedrige Dosis Beta-Glucan und die niedrige Dosis ungleich Null ist, wobei die erste und zweite Phase 2 bis 4 Monate dauern.

2. Beta-Glucan zur Verwendung gemäß Anspruch 1, **dadurch gekennzeichnet, dass** es sich um ein fungales β-(1,3/1,6)-Glucan handelt.

3. Beta-Glucan zur Verwendung gemäß Anspruch 2, **dadurch gekennzeichnet, dass** es aus Austern-Seitling hergestellt wird.

4. Beta-Glucan zur Verwendung gemäß einem der Ansprüche 1 bis 3, das frühestens 12 Monate nach Abschluss der primären onkologischen Behandlung verabreicht wird.

5. Beta-Glucan zur Verwendung gemäß einem der Ansprüche 1 bis 4, **dadurch gekennzeichnet, dass** die hohe Dosis Beta-Glucan täglich im Bereich von 600 mg bis 800 mg, vorzugsweise 700 mg, und die niedrige Dosis Beta-Glucan täglich im Bereich von 50 mg bis 300 mg, vorzugsweise 100 bis 200 mg, beträgt.

6. Beta-Glucan zur Verwendung gemäß einem der Ansprüche 1 bis 5, **dadurch gekennzeichnet, dass** die hohe Dosis während der Einnahme ein konstantes Niveau aufweist und die niedrige Dosis regelmäßig zwischen den Werten 100 und 200 mg wechselt.

7. Beta-Glucan zur Verwendung gemäß einem der Ansprüche 1 bis 6, **dadurch gekennzeichnet, dass** die erste und zweite Phase jeweils 3 Monate dauern.

8. Beta-Glucan zur Verwendung gemäß einem der Ansprüche 1 bis 7, **dadurch gekennzeichnet, dass** die erste Phase und die zweite Phase gleich lang sind.

## Revendications

1. Bêta-glucane pour utilisation dans le traitement en rémission après le traitement du cancer du sein hormono-dépendant, sous une forme adaptée à l'administration orale, administré au plus tôt 6 mois après la fin du traitement oncologique primaire, pour une administration continue en deux phases consécutives et se répétant par la suite, dans lequel, pendant la première phase, une dose élevée de bêta-glucane est administrée et dans la seconde phase, une dose faible de bêta-glucane est administrée, ladite dose élevée de bêta-glucane étant au moins deux fois supérieure à la dose faible de bêta-glucane et la dose faible étant non nulle, la première phase et la seconde phase durant de 2 à 4 mois.

2. Bêta-glucane pour utilisation selon la revendication 1, **caractérisé en ce qu'**il s'agit d'un β-(1,3/1,6)-glucane fongique.

3. Bêta-glucane pour utilisation selon la revendication 2, **caractérisé en ce qu'**il est préparé à partir de pleurote en huître (Pleurotus ostreatus).

4. Bêta-glucane pour utilisation selon l'une quelconque des revendications 1 à 3, administré au plus tôt 12 mois après la fin du traitement oncologique

5. Béta-glucane pour utilisation selon l'une quelconque des revendications 1 à 4, , **caractérisé en ce que** la dose élevée de bêta-glucane est comprise dans la plage de 600 mg à 800 mg par jour, de préférence de 700 mg, et la dose faible de bêta-glucane est comprise dans la plage de 50 mg à 300 mg par jour, de préférence de 100 à 200 mg.

6. Bêta-glucane pour utilisation selon l'une quelconque des revendications 1 à 5, **caractérisé en ce que** la dose élevée a un niveau constant pendant l'administration et la dose faible alterne régulièrement entre les valeurs de 100 et 200 mg.

7. Bêta-glucane pour utilisation selon l'une quelconque des revendications 1 à 6, **caractérisé en ce que** la première phase et la seconde phase durent 3 mois.

8. Bêta-glucane pour utilisation selon l'une quelconque des revendications 1 à 7, , **caractérisé en ce que**, la première phase et la seconde phase ont la même durée.
